(19) Europäisches Patentamt
European Patent Office
Office européen des brevets

(11) **EP 3 154 496 B1**

(12) **EUROPEAN PATENT SPECIFICATION**

(45) Date of publication and mention of the grant of the patent:
**01.05.2019 Bulletin 2019/18**

(21) Application number: **14894586.8**

(22) Date of filing: **10.06.2014**

(51) Int Cl.:
*A61F 13/84* (2006.01)   *A61L 15/46* (2006.01)
*A61F 13/511* (2006.01)

(86) International application number:
**PCT/SE2014/050702**

(87) International publication number:
**WO 2015/190957 (17.12.2015 Gazette 2015/50)**

(54) **ABSORBENT PRODUCT COMPRISING A MICROBE-INHIBITING COMPOSITION**

SAUGFÄHIGES PRODUKT MIT MIKROBENHEMMENDER ZUSAMMENSETZUNG

PRODUIT ABSORBANT CONTENANT UNE COMPOSITION ANTI-MICROBIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.04.2017 Bulletin 2017/16**

(73) Proprietor: **Essity Hygiene and Health Aktiebolag**
**405 03 Göteborg (SE)**

(72) Inventors:
• **ANDREASSEN, Petter**
**405 03 Göteborg (SE)**
• **ROMERO GÓMEZ, Vania Lizet**
**C.P. 45130 Jalisco (MX)**

(74) Representative: **Zacco Sweden AB**
**P.O. Box 5581**
**114 85 Stockholm (SE)**

(56) References cited:
WO-A1-03/004070    WO-A1-2011/047457
WO-A2-03/002698    WO-A2-2013/091894
US-A1- 2007 219 515    US-A1- 2008 249 491
US-B1- 6 803 045

• DATABASE WPI Week 199817, Derwent Publications Ltd., London, GB; Class D21, AN 1998-189143, XP055356265 & JP H1 045562 A (NOEVIR KK) 17 February 1998
• SHIN, H.T. ET AL.: 'Diaper dermatitis that does not quit' DERMATOLOGIC THERAPY vol. 18, no. 2, 2005, ISSN 1396-0296 pages 124 - 135, XP055243230
• VISSCHER, M. O.: 'Update on the use of topical agents in neonates' NEWBORN AND INFANT NURSING REVIEWS vol. 9, no. 1, 2009, ISSN 1527-3369 pages 31 - 47, XP025907358
• KATHIRVEL, K. P. ET AL.: 'Development of antimicrobial feminine hygiene products using bamboo and aloevera fibers' JOURNAL OF NATURAL FIBERS vol. 11, no. 3, 2014, ISSN 1544-0478 pages 242 - 255, XP055243214
• 'AATCC Test Method 147-201, in AATCC Technical Manual/2015' COPYRIGHT © 2014 AMERICAN ASSOCIATION OF TEXTILE CHEMISTS AND COLORISTS XP008184144

**EP 3 154 496 B1**

## Description

### TECHNICAL FIELD

The present invention relates to an absorbent product selected from a diaper, sanitary napkin, panty liner, incontinence protector, tampon, that comprises a top sheet comprising a microbe inhibiting composition.

### BACKGROUND

The urogenital area is a sensitive region with a delicate balance of moisture, fat and microorganisms, which is easily disturbed and which may cause discomfort. For example, the balance may be disturbed by extensive cleaning, the use of antibiotics or antifungal agents, and the use of absorbent products that may dry out the skin and mucous membranes. It is known that the administration of different substances to the urogenital area, such as prebiotics, probiotics, skin soothing substances etc., may improve the balance in the urogenital area and/or prevent discomfort.

The urogenital area harbors a complex microbial ecosystem comprising many different bacterial species. The microbial ecosystem of a healthy individual can be disturbed by the use of for example antibiotics. Also, microorganisms may spread from the anus to the urogenital area, thereby causing infections. This results in a disturbance of the normal microbial flora and leaves the individuals susceptible to microbial infections that cause vaginitis, urinary tract infections and ordinary skin infections.

Microorganisms commonly associated with these kinds of infections belong to the genera *Escherichia, Enterococcus, Pseudomonas, Proteus, Klebsiella, Streptococcus, Staphylococcus, Gardnerella* and *Candida.*

*Staphylococcus aureus* is the most common cause of minor skin infections, such as boils or abscesses, as well as more serious post-operative wound infections. Treatment involves drainage and this is usually sufficient for minor lesions, but antibiotics may be given in addition when the infection is severe and the patient has fever.

One way to reduce the problems with the kind of infections described above is to have a good personal hygiene. However, excessive use of cleaning agents not only decreases the amount of harmful microbes, but can harm the beneficial microbial flora, again render it susceptible for pathogenic species to colonize and cause infections.

WO 03/004070 A1 describes a disposable diaper having a film forming composition on the top sheet that comprises a lipid carrier and zinc oxide.

In view of the prior art there is still a need for absorbent products with an improved inhibitor effect on unwanted microorganisms.

## SUMMARY

It is an object of the present invention to provide an absorbent product with an improved inhibitory effect on unwanted microorganisms. It is a further object of the present invention tc improve the distribution of a microbe-inhibiting composition in the final products so that an efficient microbe-inhibiting system can be obtained without impairing the absorption properties in the final product.

The above defined problem is solved by an absorbent product, such as an absorbent produc in the form of a diaper, adult incontinence protector, incontinence pad, sanitary napkin or panty liner comprising a liquid permeable top sheet, wherein a microbe-inhibiting compositio is applied on at least a portion of the top sheet and wherein the microbe-inhibiting composition comprises a lipid carrier, Aloe Vera and zinc oxide. The microbe inhibiting composition of zinc oxide and Aloe Vera gives an unexpected synergistic effect in inhibition of pathogenic microorganisms, and an absorbent product comprising this microbe inhibiting composition has an enhanced probiotic and/or prebiotic effect.

Extracts from *Aloe Vera* are widely used in the cosmetics and alternative medicine industries being marketed as variously having rejuvenating, healing, or soothing properties. *Aloe vera* i for example used on facial tissues where it is promoted as a moisturizer and anti-irritant to reduce chafing of the nose. Cosmetic companies commonly add derivatives from *Aloe vera* to products such as makeup, tissues, moisturizers, soaps, sunscreens, incense, shaving cream, or shampoos. A review of academic literature notes that its inclusion in many hygiene products is due to its "moisturizing emollient effect". The Aloe Vera may be added to the lipi carrier as a liquid or in a dry state.

Zinc oxide is commonly used as a sun blocking ingredient. Zinc oxide has a long history of safe use. It is not irritating and compatible with sensitive skin. In fact, zinc oxide is a skin protectant and anti-irritant, and is widely used in treating various forms of dermatitis/skin irritation. The zinc oxide which is suitable for use includes those inorganic white and yellowish white powders that conform to the formula ZnO.

According to one embodiment is the zinc oxide in the form of a powder. Powders are very difficult to handle in dry processes due to dusting problems. Therefore, an advantage by mixing the powder of the zinc oxide with a lipid carrier is that contamination of both the process equipment and the products with the powder of the zinc oxide is avoided.

According to one embodiment is the lipid carrier selected from petroleum derived lipid, synthetic lipid, animal or plant derived lipid and is in the form of a fat, oil, or a wax or a mixture thereof.

The lipid carrier may be petrolatum. Petrolatum is a petroleum based hydrocarbon with carbon numbers mainly higher than 25. Synonym to petrolatum is petroleum jelly, white petrolatum, soft paraffin or multi-hydrocarbon and the Brand name Vaseline is often used. Petrolatum is hydrophobic and is originally promoted as a topical ointment for its healing properties. It is widely used as a skin protectant, and is commonly used in the cosmetic skin care area.

According to one embodiment is the concentration of the lipid carrier 80.0 - 96.5 weight percent, the concentration of the zinc oxide 1.5 - 10.0 weight percent and the concentration of the Aloe Vera 2.0 - 10.0 weight percent.

According to one embodiment is the concentration of the lipid carrier 88.0 - 96.0 weight percent, zinc oxide 1.5 - 4.0 weight percent and Aloe Vera 2.5 - 8.0 weight percent.

According to yet another embodiment is the concentration of the lipid carrier 92.0 weight percent or less, zinc oxide at least 2.5 weight percent and Aloe Vera at least 5.5 weight percent. The concentration of the lipid carrier may be 82.0 - 92.0 weight percent, zinc oxide 2.5 - 8.0 weight percent and Aloe Vera 5.5 -10 weight percent.

According to yet another embodiment is the micro-inhibiting composition a substantially homogenous mix of the zinc oxide, Aloe Vera and the lipid carrier.

According to one embodiment is said microbe-inhibiting composition applied in an intermittent pattern with first regions coated with the microbe-inhibiting composition and second regions free of the microbe-inhibiting composition. The top sheet, for example the nonwoven in baby diapers, must be pervious to liquids and has therefore usually been prepared to be hydrophilic. The further finishing with a hydrophobic microbe-inhibiting composition may therefore reduce or significantly impair the transportation of liquid through the web into the absorbing materials. It is therefore advantageous, that the microbe-inhibiting composition covers only 1 - 20 % of the total surface area of the top sheet, or 1 - 10 % of the total surface area of the top sheet, or 1 - 5 % of the total surface area of the top sheet.

According to one embodiment is the pattern of the first regions with the microbe-inhibiting composition at least two stripes extending generally parallel in relation to each other in the longitudinal direction of the absorbent product. By using a lipid carrier, which is hydrophobic, is it also possible for the microbe-inhibiting composition to function as a liquid barrier making it possible to steer the liquid in a certain direction. According to one embodiment, is the microbe-inhibiting composition applied to the top sheet in stripes in the longitudinally direction of the absorbent product. The longitudinal stripes across the length of the diaper wi function as liquid barriers in the longitudinal direction of the absorbent product and thereby decrease the risk of the liquid to be spread in the cross direction of the product.

The pattern of the first regions with the microbe-inhibiting composition may be two stripes extending generally parallel in the longitudinal direction of the absorbent product wherein the length in the transverse direction of the absorbent product from the longitudinal centre line o the absorbent product, is the same for each stripe. The length in the transverse direction of the absorbent product from the longitudinal centre line to each stripe may be between 7 mm to 15 mm. It is advantageous that the region in the the wetting zone, is free from microbe-inhibiting substance since the lipid carrier makes the microbe-inhibiting composition hydrophobic.

According to yet another embodiment is the pattern of the first regions with the microbe-inhibiting composition two stripes extending generally parallel in the longitudinal direction of the absorbent product and the distance between the two stripes in the transverse direction o the product is at least 16 mm, or at least 18 mm.

According to one embodiment has each stripe a width of 1.0 - 8.0 millimeter, or a width of 2.0 - 5.0 millimeter.

The absorbent product, according to one embodiment, has a liquid impermeable backsheet, a liquid pervious topsheet and an absorbent core enclosed there between.

DESCRIPTION OF THE DRAWING

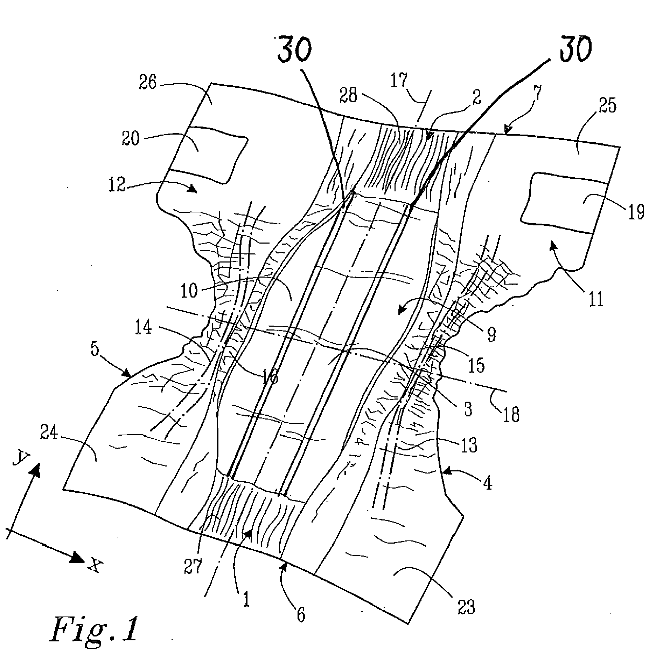

Fig. 1 is a plan view of a diaper according to the present invention.

DETAILED DESCRIPTION

As an example of an absorbent product, a diaper will be described. The diaper has longitudinal direction - the y-direction - with a longitudinal centre line 17 and a transvers direction - the x-direction - with a transverse centre line 18. In Fig. 1, a diaper is show which comprises a front and a rear end portion 1,2, a crotch portion 3 lying there between which, in use of the diaper, is intended to be brought between the legs of the user. The diaper further has longitudinal edges 4,5, a front transverse edge 6 and a rear transvers edge 7, a lower liquid impermeable back sheet 8, a first liquid permeable top sheet 10 and a absorbent core 9 placed there between. Further, the diaper possesses longitudinal leakag barriers 15,16 which run substantially parallel to the longitudinal centre line 17 of the diaper and near the longitudinal edges of the absorbent core 9. The leakage barriers 15,16 contai elastic and in the figure are wrinkled together at least in their centre portions.

Side flaps 11,12 extend at the sides outside the leakage barriers 15,16 and possess at least one longitudinal elastic elements 13,14 in the crotch portion 3 along their free edges. The elastic elements 13,14 serve as leg elastic upon use of the article and provide an extr leakage barrier.

The diaper also comprises fastening means 19,20 (folded towards the topsheet of the diaper in Figure 1) in the form of hook-and-loop type fastening means; in alternative embodiments tape with glue or other similar means may also be used. The diaper may also posses reception surfaces for said fastening means (not shown in the Figures) which are specially adapted for said fastening means.

The front 23, 24 and rear 25, 26 end regions of the side flaps 11,12 are seen at the front and rear end portion 2 of the diaper. The diaper shown in Figs. 1 has front and rear waist elastic 27,28 along at least a part of the front 23,24 and rear 25,26 end regions of the sid flaps 11,12. As shown in Figs. 1, the waist elastic 27, 28 can be located on both transvers edges 6,7 of the diaper. Front and rear waist elastic 27, 28 extends along roughly a third c the length of each transverse edge 6,7. In other embodiments, only a rear waist elastic ma be present. Waist elastic may also exist which extends entirely along both transverse edge of the absorbent article.

The absorbent core 9 may comprise cellulose fibres, with mixing of superabsorbent particle or superabsorbent fibres. However, the absorbent core may be constructed of any suitabl standard material which is usually present in absorbent cores for absorbent articles such a diapers, pant-diapers, incontinence shields, panty liners and the like. The absorbent core may be constructed of more than one layer of absorbent material. Absorbent cores usuall contain layers of wadding, receiving and distribution layers, to be able to rapidly take away released liquid from the liquid-receiving top sheet 10. Each of the layers comprising the absorbent core may contain superabsorbents.

The liquid-impervious back sheet 8 can comprise or consist of a liquid-impermeable plastic film, a nonwoven sheet treated with liquid-resistant material, or some other flexible material layer which has the ability to resist liquid penetration. It is usually an advantage if the liquid impermeable back sheet 8 is breathable, i.e. allows the passage of water vapour through the sheet 8.

The liquid-permeable top sheet 9 may comprise a nonwoven sheet of e.g. polyethene fibres polypropene fibres or mixtures thereof, perforated films or tissue layers.

In figure 1, the microbe-inhibiting composition is applied to the top sheet in two stripes 30 extending generally parallel in the longitudinal direction of the diaper. The stripes 30 in fig have a width of 2-5 millimeters. Each stripe is located 5 to 20 mm from the longitudinal centre line 17 and preferably is the length from the longitudinal centre line 17, the same for each stripe. It is advantageous that the region in the longitudinal centre of the diaper, the wetting zone, is free from microbe-inhibiting substance since the lipid carrier makes the microbe inhibiting composition hydrophobic. The microbe-inhibiting composition in figure 1 is covering 1 - 5 % of the total surface area of the top sheet.

It is also possible to have the microbe-inhibiting composition applied to the top sheet in mor than two stripes, or in another pattern. The microbe-inhibiting composition may also be applied on elastic members, belts etc., which during use of the product is in contact with the skin of the wearer via for example the liquid pervious top sheet.

EXPERIMENTS

The bacterial growth of Staphylococcus aureus, E.coli and Aspergillus niger was measured on three different nonwoven materials. The method used was the Antibacterial Activity Assessment of Textile Materials: Parallell Streak Method, AATCC Test Method 147-2004. It is a method to determine antibacterial activity of diffusible antimicrobial agents on treated textile materials.

Tested materials

1. Nonwoven comprising carrier substance of petrolatum and Aloe Vera.
2. Nonwoven comprising carrier substance of petrolatum and zinc oxide
3. Nonwoven comprising carrier substance of petrolatum, zinc oxide and Aloe Vera.

The nonwoven is made of polypropylene and has a surface weight of 12.5 gsm and the whole surface of the nonwoven material is covered with the microbe-inhibiting composition. The amount of Aloe Vera in the microbe-inhibiting composition for material 1 is 5.5 weight percent and the amount of petrolatum is 94.5 weight percent. The weight percent of zinc oxide in the microbe-inhibiting composition for material 2 is 2.5 and the petrolatum is 97.5 weight percent. The weight percent of Aloe Vera in the microbe-inhibiting composition for material 3 is 5.5, the weight percent of zinc oxide in the microbe-inhibiting composition for material 3 is 2.5 and the weight percent of petrolatum is 92.

Procedure

Dispense sterilized nutrient agar by pouring 15 ml into each standard flat bottomed petri dish Allow agar to gel firmly before inoculating. Prepare inoculum by transferring 1.0 ml of 24 broth culture into 9.0 ml of sterile distilled water contained in a test tube. Mix well using appropriate agitation. Using a 4 mm inoculating loop, load one loopful of the diluted inoculun and transfer to the surface of the sterile agar plate by making five streaks 60 mm in length, spaced 10 mm apart covering the central area of a standard petri dish without refilling the loop. Gently press the test nonwoven transversely across the five inoculum streaks to ensun intimate contact with the agar surface. Incubate at 37 degree Celsius for 24 hours.

Evaluation

Examine the incubated plates for interruption of growth along the streaks of inoculum beneath the nonwoven and for a clear zone of inhibition beyond its edge.

The average width of a zone of inhibition along a streak on either side of the nonwoven is calculated using the following equation:

$$W = (T-D)/2$$

where:

W= width of clear zone in inhibition in mm
T= total diameter of nonwoven and clear zone in mm
D= diameter of the nonwoven in mm

Result - Width of clear zone of inhibition in mm

| Material | S. aureus | E.coli | A. niger |
|----------|-----------|--------|----------|
| 1. | 1 | 1 | 0 |
| 2. | 4 | 3 | 1 |
| 3. | 4 | 4 | 2 |

The width of the clear zone of inhibition of E.coli and A.niger was longer for material 3 than for material 1 or material 2. The microbe-inhibiting composition on material 3 comprised petrolatum, zinc-oxide and Aloe Vera. So, an unexpected increased inhibition was obtained when a combination of zinc oxide and Aloe Vera was applied to the nonwoven material compared to only Aloe Vera or only zinc oxide.

**Claims**

1. An absorbent product selected from a diaper, adult incontinence protector, incontinence pad, sanitary napkin or panty liner comprising a liquid permeable top sheet (10), wherein a microbe-inhibiting composition is applied on at least a portion of the top sheet (10) **characterized in that** the microbe-inhibiting composition comprises a lipid carrier, Aloe Vera and zinc oxide.

2. An absorbent product according to claim 1, wherein the zinc oxide is in the form of a powder.

3. An absorbent product according to claim 1 or 2, wherein the lipid carrier is selected from petroleum derived lipid, synthetic lipid, animal or plant derived lipid and is in the form of a fat, an oil, or a wax or a mixture thereof.

4. An absorbent product according to any of claims 1-3, wherein the lipid carrier is petrolatum.

5. An absorbent product according any of claims 1-4, wherein the concentration of the lipid carrier is between 80.0 - 96.0 weight percent, the concentration of the zinc oxide is 1.5 - 10.0 weight percent and the concentration of the Aloe Vera is 2.0 - 10.0 weight percent.

6. An absorbent product according to any of claims 1-5, wherein the concentration of th lipid carrier is 82.0 - 92.0 weight percent, the concentration of the zinc oxide is 2.5 - 8.0 weight percent and the concentration of the Aloe Vera is 5.5 - 10.0 weight percent

7. An absorbent product according to any of claims 1-6, wherein the micro-inhibiting composition is a substantially homogenous mix of the zinc oxide, Aloe Vera and the lipid carrier.

8. An absorbent product according to any of claims 1 - 7, wherein said microbe-inhibiting composition is applied in an intermittent pattern with first regions coated with the microbe-inhibiting composition and second regions free of the microbe-inhibiting composition.

9. An absorbent product according to any of claims 1-8, wherein the microbe-inhibiting composition is covering 1 - 20 % of the total surface area of the top sheet.

10. An absorbent product according to any of claims 1-9, wherein the microbe-inhibiting composition is covering 1 - 10 % of the total surface area of the top sheet.

11. An absorbent product according to any of claims 1-10, wherein the microbe-inhibiting composition is covering or 1 - 5 % of the total surface area of the top sheet.

12. An absorbent product according to any of claims 8 to 11 wherein the pattern of the first regions with the microbe-inhibiting composition are at least two stripes (30) extending generally parallel in relation to each other in the longitudinal direction of the absorbent product.

13. An absorbent product according to claim 12 wherein the pattern of the first regions with the microbe-inhibiting composition are two stripes (30) extending generally parallel in the longitudinal direction of the absorbent product and that the distance between the two stripes in the cross direction of the product is at least 16 mm, or at least 18 mm.

14. An absorbent product according to claim 12 or 13 wherein each stripe (30) has a width of 1.0 - 8.0 millimeter.

15. An absorbent product according to any of claims 12-14, wherein each stripe (30) has a width of 2.0 - 5.0 millimeter.

**Patentansprüche**

1. Saugfähiges Produkt, ausgewählt aus Windel, Erwachseneninkontinenzprotektor, Inkontinenzeinlage, Binde oder Slipeinlage, umfassend eine flüssigkeitsdurchlässige Decklage (10), wobei eine mikrobenhemmende Zusammensetzung auf mindestens einem Teil der Decklage (10) aufgebracht ist, **dadurch gekennzeichnet, dass** die mikrobenhemmende Zusammensetzung einen Lipidträger, Aloe Vera und Zinkoxid umfasst.

2. Saugfähiges Produkt gemäß Anspruch 1, wobei das Zinkoxid in der Form eines Pulvers vorliegt.

3. Saugfähiges Produkt gemäß Anspruch 1 oder 2, wobei der Lipidträger aus aus Erdöl abgeleitetem Lipid, synthetischem Lipid, aus Tieren oder Pflanzen abgeleitetem Lipid ausgewählt ist und in der Form eines Fetts, eines Öls oder eines Wachses oder einer Mischung davon vorliegt.

4. Saugfähiges Produkt gemäß einem der Ansprüche 1-3, wobei der Lipidträger Vaseline ist.

5. Saugfähiges Produkt gemäß einem der Ansprüche 1-4, wobei die Konzentration des Lipidträgers zwischen 80,0-96,0 Gewichtsprozent liegt, die Konzentration des Zinkoxids 1,5-10,0 Gewichtsprozent beträgt und die Konzentration des Aloe Vera 2,0-10,0 Gewichtsprozent beträgt.

6. Saugfähiges Produkt gemäß einem der Ansprüche 1-5, wobei die Konzentration des Lipidträgers 82,0-92,0 Gewichtsprozent beträgt, die Konzentration des Zinkoxids 2,5-8,0 Gewichtsprozent beträgt und die Konzentration des Aloe Vera 5,5-10,0 Gewichtsprozent beträgt.

7. Saugfähiges Produkt gemäß einem der Ansprüche 1-6, wobei die mikrobenhemmende Zusammensetzung ein im Wesentlichen homogenes Gemisch von Zinkoxid, Aloe Vera und dem Lipidträger ist.

8. Saugfähiges Produkt gemäß einem der Ansprüche 1-7, wobei die genannte mikrobenhemmende Zusammensetzung in einem intermittierenden Muster mit ersten Bereichen, beschichtet mit der mikrobenhemmenden Zusammensetzung, und zweiten Bereichen, die frei von der mikrobenhemmenden Zusammensetzung sind, aufgetragen wird.

9. Saugfähiges Produkt gemäß einem der Ansprüche 1-8, wobei die mikrobenhemmende Zusammensetzung 1-20 % der gesamten Oberfläche der Decklage bedeckt.

10. Saugfähiges Produkt gemäß einem der Ansprüche 1-9, wobei die mikrobenhemmende Zusammensetzung 1-10 % der gesamten Oberfläche der Decklage bedeckt.

11. Saugfähiges Produkt gemäß einem der Ansprüche 1-10, wobei die mikrobenhemmende Zusammensetzung 1-5 % der gesamten Oberfläche der Decklage bedeckt.

12. Saugfähiges Produkt gemäß einem der Ansprüche 8 bis 11, wobei das Muster der ersten Bereiche mit der mikrobenhemmenden Zusammensetzung mindestens zwei Streifen (30) sind, die sich im Allgemeinen parallel in Relation zueinander in der Längsrichtung des saugfähigen Produkts erstrecken.

13. Saugfähiges Produkt gemäß Anspruch 12, wobei das Muster der ersten Bereiche mit der mikrobenhemmenden Zusammensetzung zwei Streifen (30) sind, die sich im Allgemeinen parallel in Relation zueinander in der Längsrichtung des saugfähigen Produkts erstrecken, und wobei der Abstand zwischen den zwei Streifen in der Querrichtung des Produkts mindestens 16 mm oder mindestens 18 mm beträgt.

14. Saugfähiges Produkt gemäß Anspruch 12 oder 13, wobei jeder Streifen (30) eine Breite von 1,0-8,0 Millimeter aufweist.

15. Saugfähiges Produkt gemäß einem der Ansprüche 12-14, wobei jeder Streifen (30) eine Breite von 2,0-5,0 Millimeter aufweist.

**Revendications**

1. Produit absorbant choisi entre une couche-culotte, une protection pour incontinent adulte, une serviette pour incontinent, une serviette hygiénique ou un protège-slip comprenant une feuille supérieure perméable aux liquides (10), dans lequel une composition inhibant les microbes est appliquée sur au moins une partie de la feuille supérieure (10), **caractérisé en ce que** la composition inhibant les microbes comprend un vecteur lipidique, de *l'Aloe vera* et de l'oxyde de zinc.

2. Produit absorbant selon la revendication 1, dans lequel l'oxyde de zinc est sous la forme d'une poudre.

3. Produit absorbant selon la revendication 1 ou 2, dans lequel le vecteur lipidique est choisi entre un lipide issu de pétrole, un lipide synthétique et un lipide issu d'un animal ou d'une plante et est sous la forme d'une matière grasse, d'une huile ou d'une cire ou d'un mélange de celles-ci.

4. Produit absorbant selon l'une quelconque des revendications 1-3, dans lequel le vecteur lipidique est du pétrolatum.

5. Produit absorbant selon l'une quelconque des revendications 1-4, dans lequel la concentration du vecteur lipidique est comprise entre 80,0 et 96,0 pour cent en poids, la concentration de l'oxyde de zinc est de 1,5-10,0 pour cent en poids et la concentration de l'*Aloe vera* est de 2,0-10,0 pour cent en poids.

6. Produit absorbant selon l'une quelconque des revendications 1-5, dans lequel la concentration du vecteur lipidique est de 82,0-92,0 pour cent en poids, la concentration de l'oxyde de zinc est de 2,5-8,0 pour cent en poids et la concentration de l'*Aloe vera* est de 5,5-10,0 pour cent en poids.

7. Produit absorbant selon l'une quelconque des revendications 1-6, dans lequel la composition inhibant les microbes est un mélange pratiquement homogène de l'oxyde de zinc, de *l'Aloe vera* et du vecteur lipidique.

8. Produit absorbant selon l'une quelconque des revendications 1-7, dans lequel ladite composition inhibant les microbes est appliquée suivant un motif discontinu avec des premières zones revêtues de la composition inhibant les microbes et des secondes zones dépourvues de la composition inhibant les microbes.

9. Produit absorbant selon l'une quelconque des revendications 1-8, dans lequel la composition inhibant les microbes recouvre 1-20 % de la surface totale de la feuille supérieure.

10. Produit absorbant selon l'une quelconque des revendications 1-9, dans lequel la composition inhibant les microbes recouvre 1-10 % de la surface totale de la feuille supérieure.

11. Produit absorbant selon l'une quelconque des revendications 1-10, dans lequel la composition inhibant les microbes recouvre 1-5 % de la surface totale de la feuille supérieure.

12. Produit absorbant selon l'une quelconque des revendications 8 à 11 dans lequel le motif des premières zones comprenant la composition inhibant les microbes est constitué d'au moins deux bandes (30) s'étendant généralement parallèlement les unes par rapport aux autres dans la direction longitudinale du produit absorbant.

13. Produit absorbant selon la revendication 12 dans lequel le motif des premières zones comprenant la composition inhibant les microbes est constitué de deux bandes (30) s'étendant généralement parallèlement dans la direction longitudinale du produit absorbant et la distance entre les deux bandes dans la direction transversale du produit est d'au moins 16 mm ou d'au moins 18 mm.

14. Produit absorbant selon la revendication 12 ou 13 dans lequel chaque bande (30) a une largeur de 1,0-8,0 millimètres.

15. Produit absorbant selon l'une quelconque des revendications 12-14, dans lequel chaque bande (30) a une largeur de 2,0-5,0 millimètres.

*Fig.1*

**EP 3 154 496 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03004070 A1